(19) Europäisches Patentamt
European Patent Office
Office européen des brevets

(11) Publication number: 0 465 715 A1

# (12) EUROPEAN PATENT APPLICATION

(21) Application number: **90115425.2**

(22) Date of filing: **10.08.90**

(51) Int. Cl.$^5$: **G01N 33/574**, C12Q 1/68, G01N 33/58

(30) Priority: **13.07.90 US 552409**

(43) Date of publication of application: **15.01.92 Bulletin 92/03**

(84) Designated Contracting States: **AT BE CH DE DK ES FR GB GR IT LI LU NL SE**

(71) Applicant: **AMDL, INC**
**27281 Las Ramblas, Suite 200**
**Mission Viejo, California 92691(US)**

(72) Inventor: **Guerrero, Robert R.**
**814 S. Euclid Avenue**
**Pasadena, California 91106(US)**

(74) Representative: **WILHELMS, KILIAN & PARTNER Patentanwälte**
**Eduard-Schmid-Strasse 2**
**W-8000 München 90(DE)**

## (54) An in vitro method and probe for detecting the presence of the ring shaped particle and malignancy in humans and animals.

(57) An in vitro method and probe for determining the presence of the ring shaped particle (RSP) tumor marker and for detecting the presence of malignancy in humans and animals comprises capturing the marker out of a biological fluid containing cancerous cells onto a substrate and detecting the presence of the RSP tumor marker on the substrate as an indicator of the presence of the malignancy. One preferred method involves using an anti-RSP antibody (rspAb) selected from one or more of the monoclonal, polyclonal and affinity purified antibodies to capture the tumor marker from a sample of a biological fluid such as blood serum, sputum, urine, irrigation fluids, etc.. The tumor marker is captured onto a substrate with an rspAb and its presence on the substrate is detected using common detection systems, such as color development techniques. In one preferred form, the tumor marker is detected on the substrate by reaction with a tRNA-photobiotin-streptavidin-alkaline phosphatase probe. Another preferred method involves using a transfer ribonucleic acid (tRNA) probe which is specific to the aminoacyl transfer RNA synthetase reactive site on the marker and detecting the presence of the RSP by color development techniques using a tRNA-photobiotin-streptavidin-alkaline phosphatase probe.

EP 0 465 715 A1

## Field of the Invention

The present invention relates to cancer detection and, more particularly, to methods and probes for determining the presence of a tumor marker for reliably indicating the presence of cancer cells.

## Background of the Invention

Cancer is a large group of diseases characterized by uncontrolled cellular growth and spread of abnormal cells. If the spread is not controlled or checked, it results in death. However, many cancers can be cured if detected early and treated promptly. The incidence of cancer is increasing. In 1988 about 985,000 people will be newly diagnosed as having cancer in the United States alone. During the same year about 494,000 people in the United States and 4,000,000 worldwide, will die of the disease.

About 174,000 people in the U.S. alone might have been saved by earlier diagnosis, prompt treatment, and proper monitoring. Early detection of cancer greatly increases the potential of cure. Detection of cancer in a localized area, possibly with diagnosis early in the growth of the tumor, permits the use of more effective therapy, and improves the chance for recovery. Treatment by surgery, chemotherapy or radiation therapy requires post-treatment monitoring to ensure that all vestiges of the malignant growth have been eliminated.

Over the past 15 years, a number of tumor-associated proteins have been discovered, some of which have been considered for use as tumor markers. Many of these tumor markers are uniquely associated with specific tumors. As a result, their presence can be used to reliably detect these tumors. Because the tumor marker may be more readily identified than the tumor itself, the marker makes earlier, more reliable detection of the tumor possible. Some of the better known tumor markers are (a) carcinoembryonic antigen (CEA) for cancers of the colon, lung, stomach, and pancreas; (b) alpha-fetoprotein (AFP) for testicular and liver cancers; and, (c) a recently approved tumor marker for breast cancers (CA-549).

One tumor marker has been identified which, unlike those just mentioned, appears to be a universal tumor marker. This tumor marker, in the form of submicroscopic ring-shaped particles (RSP) when viewed by electron microscopy is released into surrounding tissue and body fluids from cancer cells, but is not released in detectable quantities by normal cells. RSP, also known as prosome, proteasome, miniparticles, LAMP, macropain, RNA proteolipid and ring shaped miniparticles, have been found to be released by the broadest spectrum of human tumors as well as in tumors of other mammalian species and by malignant cell lines. In vitro and in vivo studies indicate that the release of RSP by malignant cells is a property of the carcinogenesis process and is independent of species, tumor type or tumor location in the organism. Further, this tumor marker is released in proportion to the tumor burden and is released 4 to 10 months before relapse diagnosis is possible based on overt clinical symptoms. Therefore, unlike the other markers, the RSP is a universal tumor marker. As such, RSP can be used to detect the presence of any cancer at an early stage, making the prospect for a cure significantly more promising. The RSP is also able to monitor the effectiveness of treatment in cancer patients. The reduction in the level of RSP in the serum can be used as an indicator of successful treatment. Since all malignant cells tested so far in tissue culture release RSP, and cells made malignant in tissue culture following treatment with carcinogens also release RSP, RSP can be used as the end point for a new family of short-term test systems for identifying carcinogens as well.

Early research resulted in a fluorescence assay system for detecting the DNA component in the RSP. This technique was used successfully with vaginal irrigation fluids to screen women for cervical cancer. In U,S. Patent No. 3,798,131 - Rounds et al, the technique is disclosed for binding a fluorescent dye to the DNA component of RSP and reading the increased light emission (fluorescence intensity) as an indirect measure of the RSP concentration. Unfortunately, this technique must be modified for quantitatively measuring RSP in different body fluids to account for such variables as dilution and variable volumes. Moreover, the fluorescence assay requires specialized equipment even for qualitative analysis and, therefore, does not readily lend itself to local, much less home, use or to incorporation into test kits for use in physician's offices.

The RSP tumor marker was first described in about 1970 by Dr. Donald Rounds as a growth modifying factor found in culture medium conditioned by three different human malignant cell lines (HeLa, derived from a cervical carcinoma; KB, derived from a nasopharyngeal carcinoma; and CMP, derived from an adenocarcinoma from the colon). The three cell lines excreted a common factor that modified the growth of normal human skin fibroblasts in vitro. At low concentrations it stimulated cell division. At higher concentrations it arrested cell division and, at the highest concentration, caused cell death. Antigenically, the factor was found to be the same from all three malignant cell lines.

Although much remains unknown about the function of RSP in living organisms, the physical and biochemical properties of the factor have been characterized. Using ultracentrifugation and electron microscopy procedures, it was demonstrated that the factor consisted of particles with a ring configuration. Electron micrographs showed that the rings were made up of 6-7 subunits, The rings were found singly or occasionally in stacks of 4 rings, appearing as a rectangular particle when viewed from the side. The rings were 10 to 12 nanometers in diameter and contained 3-5 ug DNA and 8-15 ug RNA per 100 ug protein. Unpublished studies with fat-soluble stains also suggested the presence of lipid(s), but these were not characterized. The RSP size and shape and number of subunits is identical in all living organisms reported to date ranging from bacteria to yeast, plants, sea urchins, insects, amphibians, reptiles, birds and mammals, including humans. RSP is a high molecular weight nucleoprotein with multicatalytic proteinase activity. It has a sedimentation coefficient of about 20S, a molecular mass of 650-750 kDa and consist of at least 13 nonidentical polypeptides with molecular weights of 21-31 kDa and isoelectric points of 3-10. They also have an RNA component comprised of about 70-80 nucleotides and a lesser DNA component. The RSP are found in the cytoplasm as well as the nucleus of the cell and they are released extracellularly by malignant cells. They have been reported to break down intracellular proteins, to regulate mRNA translation and pre-tRNA processing, and to have aminoacyl tRNA synthetase activity. They have been reported to be associated with heat shock proteins and with specific small RNA species. As such, these particles clearly play an important role in a variety of intracellular processes.

Early studies during the mid-1970's provided evidence that RSP was selectively released by tumor tissues. Minced tumor biopsy tissues from lung, colon, breast, skin, kidney, and eye released significant amounts of RSP in maintenance medium following a 24 hour incubation at body temperature, while similar preparations of normal skin and tongue muscle released no detectable levels of RSP. Double blind studies of the RSP release by incubated biopsy tissue from 146 human skin specimens ranging in pathology from normal to invasive cancer found that none of 20 normal skin biopsies and none of 10 verruca vulgaris (common wart) biopsies released significant amounts of RSP, while 12 of 36 (33%) specimens of the benign tumor, seborrheic verruca, and 5 of 13 (38%) specimens of the premalignant tumor, actinic keratosis, released significant levels of RSP. The studies also found that 100% of 52 basal cell carcinomas and 100% of 15 squamous cell carcinomas released significant amounts of RSP. The range of RSP values for the invasive squamous cell carcinomas were higher than the RSP range for the non-invasive basal cell carcinomas.

In similar double blind studies with biopsies from the human uterine cervix it was observed that 16/21 (76%) biopsies from presumably normal regions of abnormal cervices showed little or no detectable RSP released into maintenance medium. The remaining five tissue specimens released low levels of RSP during a 24 hour incubation period. Pooled dysplasia and condyloma specimens showed that 10/18 (56%) released insignificant levels of RSP while 8/18 of these specimens released moderate levels of RSP. In contrast, 13/14 (93%) carcinoma in situ and 16/16 (100%) invasive carcinoma specimens released significant levels of RSP. Furthermore, it was observed that RSP was released into the vaginal pool by cervical tissues in situ. Analyses of vaginal irrigation fluids indicated that 276/284 (97%) of normal cervices released none or an insignificant level of RSP, as did 115/124 (93%) of women with various grades of dysplasia. Specimens from women with carcinoma in situ showed that 17/29 (58%) were negative in terms of RSP release. Of these, 17/19 (89.5%) were under the age of 38, an age range where, when left untreated, the lesions revert back to normal in 90% of the cases as documented in two 15 year studies conducted in Denmark. In the case of cervical carcinoma, 10/10 (100%) of vaginal irrigation specimens showed large levels of RSP.

In a further study, a nucleolar antigen extracted from the nucleoli of a variety of cancer cells was observed to consist of "miniparticles" having an outside diameter of 11.3 nm and an inner diameter of 2.9 nm, containing 8 subunits and having the overall morphologic appearance of the RSP. This antigen was found to be present in carcinomas of the bladder, brain, colon, esophagus, liver, lung, prostate, skin, stomach and thyroid, 3 kinds of sarcomas, 4 kinds of lymphomas and 6 cultured cell lines of malignant origin. The antigen was absent in 17 normal tissue types, 4 benign tumors, 7 types of inflammatory diseases and 2 cultured cell lines of normal origin.

These observations regarding RSP release from human tumor cells had been demonstrated in an earlier study of the release of RSP into culture medium by 21 mammalian cell lines in vitro. It was found that all but one cell line released some RSP (detectable with electron microscopy). Therefore, RSP appeared to be a ubiquitous phenomenon. However, the amount released was dependent on the type or origin of the cell line. All of the cells of tumor origin (9/9) and cells infected with oncogenic viruses (3/3) released significant amounts of RSP, while 6/10 cell lines of normal origin released very low levels of RSP.

The relative amount of RSP released by tumor tissues appears to be related to the degree of their malignancy. This was well demonstrated in studies conducted with $C_3$H10T1/2 mouse cells. Such cells are

used in a widely accepted in vitro assay for identifying carcinogens. When exposed to carcinogens, these cells undergo clearly definable morphological and physiological changes which include: (a) no change (normal contact inhibition), (b) Type I colonies with marginal contact inhibition, (c) Type II colonies with low contact inhibition and (d) Type III colonies with no evidence of contact inhibition. When implanted into $C_3$H10T1/2 mice, only cells from Type II and Type III colonies will grow into malignant tumors. Normal cells and cells from Type I colonies will not form tumors. Further, the cells from Type III colonies are more virulent than those from Type II colonies since they form more and larger malignant tumors in host animals than cells from Type II colonies. It was demonstrated that only cells from Types II and III colonies release RSP while normal cells and those from Type I colonies do not. In addition it was demonstrated that RSP can be detected in medium from $C_3$H10T1/2 cells after only 3 weeks following treatment with the carcinogen, Ethylmethanesulfonate, in contrast to the usual 8 weeks required for the normal assay end point.

Another study demonstrated that notwithstanding that the tracheal epithelium of vitamin A deficient hamsters gives rise to squamous cell metaplasia and hyperplasia in organ culture, tracheas from vitamin A deficient Syrian hamsters do not release RSP. While the model closely mimics the carcinogenesis process and is used to study the carcinogenesis process and the antineoplastic properties of vitamin A analogs, the lesions revert to normal when implanted into syngeneic hamsters and are therefore not malignantly transformed. Thus, the failure to release RSP accurately reflected the true benign condition of the tissues.

There is strong evidence that RSP release is quantitatively linked to tumor burden and thus has predictive value. When cells from a mouse melanoma cell line are infused intravenously into Bl6FIO mice, they form small tumors throughout the body. The pigmented feature of these tumors permits reliable scoring of the number of tumors in lung tissue, as an indicator of the tumor burden in the host animals. It was observed that minced lung tissue from mice infused with melanoma cells released RSP into culture medium in proportion to the number of tumors recorded for the same lung tissue. Furthermore, it was observed that RSP release could be detected before the lung had formed obviously visible tumors. As further evidence that RSP release is related to tumor burden, a study was made of the RSP levels in the serum of 8 breast cancer patients whose tumor tissue had been surgically removed by radical mastectomy. Serum RSP content fell rapidly from the time of surgery to undetectable levels of RSP by the 10th post-surgical day. The serum RSP levels remained negative in these women up to 32 days post-surgery (the duration of the study).

In a subsequent study, an RNA-proteolipid material, having characteristics suggesting that the RNA-proteolipid was RSP, was observed to be released from malignant cells into their surrounding culture medium as well as in the serum of cancer patients. This material was detected in the serum of 99 patients representing 23 different types of cancer. In comparison, it was not found in the serum of 74 patients with 21 disorders unrelated to cancer. Eight cell lines of malignant origin were found to release significant amounts of the RNA-proteolipid material into their culture medium. The study reported that the amount of RSP-like RNA-proteolipid in the serum of cancer patients was directly proportional to the amount of tumor present and that surgical removal of the tumor caused a complete depletion of the RNA-proteolipid in the serum over an 8 to 10 day post-surgical period, confirming the observations with respect to breast cancer patients who underwent radical mastectomy. Moreover, where recurrence of the tumor was observed, the levels of serum RNA-proteolipid became elevated up to 10 months in advance of the clinical appearance of the tumor.

In addition, a study was conducted in which RSP release was correlated with tumor induction in hamster cheek pouches, painted 3 times per week for five weeks with 5% dimethylbenzanthracene dissolved in mineral oil. One hundred percent (32/32) normal, pretreatment cheek pouch biopsies produced insignificant levels of RSP, as did 18/18 specimens which had received 3 paintings. After the fourth painting, 5/9 (56%) showed moderately elevated RSP production, as did 9/17 (53%) of specimens harvested after the 6th painting. At this time tissue sections of the biopsies showed some hyperplasia of basal cells with increased keratinization, but no evidence of malignant cells. At the 7th painting (week 3) and thereafter, virtually all of the specimens released significant levels of RSP (e.g., 18/18 @ 7th painting, 15/15 @ 9th painting, 8/8 @ 12th painting and 12/12 @ 15th painting) and the level of RSP released plateaued after the 9th painting. Histologically, all of the treated animals developed well defined carcinomas of the cheek pouch and the lip but only after 12 weeks after the initiation of treatment. These data indicate that RSP release by chemically transformed tissue biopsies preceded histological evidence of malignant transformation by at least 9 weeks and suggests that a measurement of RSP release could be clinically useful in identifying individuals with a premalignant or asymptomatic condition.

The tumor marker first described in about 1970 and characterized as ring-shaped particles (RSP) has been observed in preparations of malignant tissues or cell lines in a number of laboratories. Current

evidence suggests that the RSP from malignant cells differs from normal cells in that they are antigenically unique and are excreted in massive amounts by malignant cells into body fluids, in contrast to no release or an insignificant release by normal cells. This latter feature permits monitoring all body fluids for evidence of cancer hidden within the body. Moreover, the observation that RSP can be detected several months prior to the clinical appearance of a recurring tumor suggests that asymptomatic tumors could be detected in human subjects at such an early stage of development that treatment could assure a high probability of a cure. The observation that RSP content was proportional to the tumor burden also suggests that cancer patients undergoing treatment could be readily monitored to assess the efficacy of treatment. To accomplish these goals a low-cost, simple, quick, sensitive and reliable procedure to detect and measure RSP levels is essential.

Summary of the Invention

It is, therefore, an object of the present invention to provide a method and probe for detecting the presence of the RSP tumor marker as a method for detecting malignancy which is low-cost, simple, easy-to-use, sensitive and reliable.

It is another object of the invention to provide a method and probe for detecting the presence of the RSP tumor marker in biological fluid by capturing the marker out of the fluids onto a substrate and subsequently detecting the presence of the RSP on the substrate.

It is still another object of the invention to provide a method and probe for detecting the presence of a malignancy comprising capturing the RSP tumor marker out of a biological fluid containing cancerous cells onto a substrate and subsequently detecting the presence of the RSP tumor marker on the substrate as an indicator of the presence of the malignancy.

It is yet another object of the invention to provide a method and probe for capturing the RSP tumor marker out of a biological fluid with anti-RSP antibodies selected from the group consisting of monoclonal antibodies, polyclonal antibodies, affinity purified antibodies and mixtures thereof.

It is still another object of the invention to provide a method and probe for capturing the RSP tumor marker out of a biological fluid with a transfer ribonucleic acid (tRNA) probe and subsequently detecting the probe presence as an indicator of the RSP presence.

It is another object of the invention to capture the RSP tumor marker out of a biological fluid onto a substrate with tRNA specific to the aminoacyl transfer RNA synthetase receptive site on the marker.

It is a further object of the invention to provide a method for detecting the presence of the RSP tumor marker in biological fluids from patients having suspected malignancies which utilizes color development techniques involving direct or indirect linking of color reactive molecules to the RSP tumor marker.

The aforesaid objects are achieved in accordance with the present invention by providing a method for the detection of the presence of RSP in biological fluids from patients who possibly have malignant tumors, which method comprises capturing the RSP in said fluid onto a substrate and detecting the presence of the RSP on the substrate. The method can be employed for detecting the presence of malignant cancerous cells in humans or animals by obtaining a body fluid sample from a human or animal to be tested, capturing the RSP in said fluid onto a substrate and detecting the presence of RSP on the substrate. In accordance with one form of the present invention the RSP is captured from the body fluid sample with anti-RSP antibodies selected from the group consisting of monoclonal antibodies, polyclonal antibodies, affinity purified antibodies and mixtures thereof. Thereafter, the presence of RSP on the substrate is detected, e.g., by using color development techniques. In accordance with another form of the present invention the RSP is captured from the body fluid sample by contacting the RSP in the body fluid sample with a labeled tRNA probe for attaching the tRNA to the aminoacyl transfer RNA synthetase reactive site on the RSP marker and detecting the presence of the synthetase molecules on the RSP.

In a preferred form of the invention, either capture and detection of the RSP or detection of already captured RSP on the substrate is accomplished using a RSP probe comprising tRNA specific to the aminoacyl transfer RNA synthetase reactive site on the RSP. Desirably, the probe is a linked molecule which, through the tRNA component, binds to the aminoacyl transfer RNA synthetase reactive site (dinucleotide fold) on the RSP and includes secondary molecules for developing visible color. One preferred probe comprises the linked molecules: tRNA-photobiotin-streptavidin-alkaline phosphatase. Another preferred probe comprises tRNA-photobiotin-avidin-glucose oxidase.

In another form of the invention, there is provided a RSP probe comprising at least one or more anti-RSP antibodies selected from monoclonal, polyclonal and affinity purified antibodies, said antibodies being linked to a probe marker whose presence is adapted to be detected as an indicator of the presence of the RSP tumor marker.

Other objects and advantages of the present invention will become apparent to those skilled in the art from the following detailed description, drawings, examples, and appended claims.

## Brief Description of the Drawings

FIGURE 1 illustrates, in diagrammatic fashion, the RSP aminoacyl tRNA synthetase receptor site and one form of an RSP probe specific to that site.

FIGURE 2 illustrates, in diagrammatic fashion, RSP bound to the reaction vessel via tRNA pretreated surfaces just prior to attachment of tRNA probes to available aminoacyl tRNA synthetase receptor sites on the RSP.

FIGURE 3 illustrates, in diagrammatic fashion, the RSP of Figure 2 following reaction with the tRNA probes at its receptor sites.

## Best Mode for Carrying Out the Invention

In accordance with the present invention there is provided a method for determining the presence of RSP tumor markers in biological fluid and for detecting the presence of malignancy in humans or animals which involves capturing the RSP marker out of the fluid onto a substrate and subsequently detecting the presence of the RSP marker on the substrate as an indicator of the presence of a malignancy. One preferred form of the invention involves the use of an anti-RSP antibody (rspAb) for capturing the RSP tumor marker in the body fluid onto a substrate. This is made possible by the affinity of the rspAb for the RSP tumor marker which causes the rspAb to link with the RSP tumor marker and, thereby, to achieve the desired capture of RSP out of the fluid. Another preferred form of the invention involves the use of a tRNA probe that binds with a very specific site, the aminoacyl transfer RNA synthetase reactive site, on RSP. Such a probe not only is capable of capturing the RSP tumor marker in the body fluid onto a substrate but also to detect the presence of the RSP on the substrate by including on the probe appropriate secondary molecules which facilitate detection, e.g., by developing visible color. The appreciation that the capture and detection of the RSP tumor marker is an effective, accurate and reliable method for detecting the presence of malignant cancerous cells in humans or animals is a direct result of the recognition that RSP are released extracellularly by cancerous cells and are, therefore, detectable in serum or other body fluids.

The ability of anti-RSP antibodies to recognize and identify RSP in biological fluids, such as human or animal body fluids, and to capture RSP out of these fluids, and the use of this method for the detection of malignant cancerous cells in human or animal body fluids, such as cancer serum, is clearly illustrated by the following Example I. The purpose of Example I is to demonstrate that an anti-RSP antibody (rspAb) can effectively capture RSP from cancer sera. Example I was conducted by recovering, via affinity purification techniques, rspAb from a former cancer patient who no longer released RSP but who still had anti-RSP antibodies. If the rspAb is effective, captured RSP will be detectable by color development techniques, such as with a tRNA probe detection system such as is disclosed more fully hereinafter.

## Example I

### 1. Affinity Purification Procedures:

a) Cyanogen bromide Sepharose 4-B resin was loaded into a column and RSP was bound to the resin. The preparation of the resin, loading of the column and binding of the antigen (RSP) are well known, standard procedures.

b) 1 ml serum from a former cancer patient was mixed with 3 ml pH 8.5 borate buffer and was then passed through the column at ambient temperature and pressure. Any rspAb in the serum reacted with and bound to the RSP antigen on the column. The non-bound serum proteins were washed from the column with pH 7.5 phosphate buffered saline (PBS). Washing was stopped when an optical density reading (0.D.) of 0.0 absorbence was achieved at 280 nm in a UV spectrophotometer.

c) The bound rspAb were eluted from the column with 2 ml aliquots of 4.0 M magnesium chloride. The 0.D. at 280 nm of each aliquot of eluent was read in the spectrophotometer with the following results:

| elution # | 0. D. |
|---|---|
| 1 | 0.04 |
| 2 | 0.02 |
| 3 | 0.02 |
| 4 | 0.01 |

d) The decreasing level of protein (rspAb) indicated that the bulk of the rspAb was in the first four elutions. These four elutions were pooled, put into a 10,000 molecular weight cut-off dialysis tubing and dialyzed against 0.85 % sodium chloride at 4° C for an initial 18 hours, and then, for a final 2.5 hours, against pH 7.5 PBS to remove the excess salt.

e) The resultant 15 ml volume of dialyzed material was concentrated to 2.5 ml with centrifugation in a AMICON Ultrafiltration cell with a PM-10 membrane.

f) The O.D. of the antibody concentrate was compared to a protein standard curve and found to be equivalent to 0.1 mg/ml protein. This material constituted the affinity purified rspAb.

2. Test System with the anti-RSP antibody:

a) The rspAb was applied to a nitrocellulose membrane (]) on a plastic stick, was blocked with serum and was then reacted with 0.1 ml of serum from a cancer patient for 10 minutes at room temperature. The membrane was blotted dry. Thereafter, it was reacted for 15 minutes with the tRNA probe detection system which will be more fully described hereinafter. The step-wise reactions are illustrated as follows:

i. rspAb is put on nitrocellulose membrane: => ]-rspAb

ii. membrane is reacted with serum of cancer patient containing RSP: => ]-rspAb-RSP

iii. RSP on membrane is reacted with tRNA probe mixture: => ]-rspAb-RSP-tRNA = formazan

The reactions resulted in the characteristic formazan gray-black spot only where the anti-RSP antibody had been applied to the membrane.

This example illustrates that the RSP was captured out of the cancer serum by the rspAb affixed on the membrane. It also illustrates that rspAb was successfully configured into a system for detecting malignancy from an aliquot of serum from a cancer patient.

Example II

The very same result as shown in Example I can be obtained using polyclonal antibodies. The following illustrates an illustrative procedure for obtaining polyclonal antibodies from rabbits.

Polyclonal anti-RSP Antibody production in rabbits

Anti-RSP antibodies are being made to further develop the antibody based cancer detection system using the following protocols:

1. Antigen (RSP) purification procedures:

a) Preparation of crude RSP extract:

The IgG and RSP from 300 ml serum from a breast cancer patient were precipitated (ppt) out of the serum by mixing with an equal volume of saturated ammonium sulfate.

b) Preparation of RSP ppt from the serum:

i. The ppt was dissolved in "standard buffer" (5OmM Tris-HCl, pH 7.5, 1mM dithiothreitol 20% v/v glycerol and 0.02% w/v sodium azide),

ii. The RSP/IgG component was re-precipitated with an equal volume of saturated ammonium sulfate. The ppt was re dissolved with standard buffer and re-precipitated and re-dissolved once again.

iii. The dissolved RSP/IgG mixture was dialyzed in a 10,000 MW cut-off dialysis membrane against phosphate buffered saline (pH 7.5) with 0.02% sodium azide at 4° C for 18 hours.

c) Biogel A-0.5m gel filtration:

The dialyzed (10ml) RSP mixture was concentrated and fractionated on a 4x8 cm column packed with Biogel A-0.5m resin. The resin was conditioned with standard buffer prior to use. The eluted fractions were tested with the tRNA probe system described more fully hereinafter to determine which fractions contained the RSP component.

d) Hydroxylapatite chromatography:

The RSP containing fractions were pooled and then dialyzed against 25mM potassium phosphate (pH 6.8) containing 1mM dithiothreitol and 20% glycerol for 18 hours at 4° C. The dialyzed RSP mixture was then concentrated and fractionated on a column packed with 25 ml of hydroxylapatite preconditioned with the same phosphate buffer. The RSP was eluted with 100 ml of a 100-150 mM phosphate buffer. The elutions were tested with the aforementioned tRNA probe system to determine which contained the RSP component.

e) DEAE Affi-Gel Blue chromatography:

The RSP-containing fractions were pooled and then dialyzed against standard buffer for 18 hours at 4° C, The dialyzed RSP mixture was then concentrated and fractionated on a column packed with 20 ml of DEAE Affi-Gel Blue equilibrated with the standard buffer. The column was washed with 50 ml of standard buffer after which the RSP were eluted with 100-130 ml of a gradient of 0-0.6 M potassium chloride. The active fractions, based on tRNA probe analysis, were pooled and then were dialyzed against standard buffer for 18 hours at 4° C. The dialyzed RSP component was concentrated with centrifugation in a AMICON cell with a PM-10 membrane to a final protein concentration of 5 mg/ml based on the O.D. at 280 nm in a UV spectrophotometer.

f) Criteria for purity:

The purified RSP was examined with electrophoresis in polyacrylamide gel under non-denaturing conditions. The result was a characteristic single band at the 600,000-675,000 mw position.

2. Immunization of Rabbits.

a) Polyclonal RSP antisera:

Antisera specific to RSP is made using a modification of the method of Winberry and Holten, "Rat Liver Glucose-6-P Dehydrogenase. Dietary Regulation of the Rate of Synthesis" The Journal of Biological Chemistry, Vol. 252, No. 21, pp. 7796-7801 (1977).

b) Immunization protocol:

i. Primary immunization with 100ug immunogen (RSP) in equal v/v with complete Freund's adjuvant. Subcutaneous (SQ) multiple site injections,
ii. Three week interval.
iii. Secondary immunization with 50 ug immunogen in equal v/v incomplete Freund's adjuvant. SQ multiple site injections.
iv. Nine to ten day interval.
v. Test bleed or production bleed depending on rspAb level from test batch of serum.
vi. Fourteen to eighteen day interval.
vii. Third immunization with 50 ug immunogen in equal v/v incomplete Freund's adjuvant. SQ multiple site injections.
viii. Nine to eleven day interval.
ix. Production bleed.

c) Purification and preparation of rspAb:

i. The IgG are separated from the other immunoglobulins by protein A-Sepharose affinity chromatography.
ii. The acid eluted IgG are neutralized and fractions that have an 0.D. greater than 0.01 at 280 nm are

pooled and concentrated with centrifugation to approximately 1 mg protein/ml in a AMICON cell with a PM-10 membrane.

iii. The resultant purified and concentrated rspAb preparation are stored at 5°C and preserved with 0.02% w/v sodium azide. This material can be used in the cancer (RSP) detection system of the present invention.

In the practice of the present invention the rspAb can be affixed to any of the commonly used substrates such as acrylic beads, membranes, latex beads, metals (i.e., steel beads, colloidal gold, etc.), glass surfaces, various polymer surfaces, and the like, to capture or anchor the RSP to a surface from human or animal body fluids. Exemplary of such body fluids are serum, urine, tears, sputum, saliva, semen, mammary gland fluids as well as lavages from accessible areas such as pulmonary, cervical, colonic lavage or from cell or tissue lysates or culture media, and the like. The captured RSP can then be detected with any of the commonly used detection systems, such as fluorescent markers, radioisotope, catalytic enzymes and bio- or chemi-luminescent tag systems or with probes such as the RNA or DNA probes developed against the nucleic acid component of the RSP or with the tRNA probe described in applicant's copending application. For example the rspAb can be linked with any of the commonly used detection systems to form a probe desirable for use in accordance with the present invention. Thus, it can be linked to fluorescent markers, such as fluorescein, rhodamine, Texas red, ethidium bromide, acridine dyes, and the like, and can be read with a spectrofluorometer, fluorescent microscope or UV spectrophotomer. In addition, it can be linked with any of the isotopes and be detected with a beta or gamma counter or with autoradiography depending on the isotope used and the configuration of the test system. The anti-RSP antibody can also be linked with biotin or photobiotin and, therefore, can be linked with avidin, streptavidin, extravidin, etc. and linked to any of the enzymes used in standard ELISA detection systems, e.g., glucose oxidase, alkaline phosphatase, horseradish peroxidase, etc. These enzyme linked antibodies can also be used with any of the systems for amplifying the color endpoints, e.g., alkaline phosphatase initiated double sequential catalytic reactions with the simultaneous formation of formazan and indigo, etc.

Probes are based on unique and specific affinities of one type of molecule for another. Examples of molecules commonly used as probes are: antigen:antibody affinities, hybridized RNA or DNA: DNA affinities, avidin:biotin affinity and the affinity of Protein A or Protein G for specific immunoglobulins. Thus, the capture of RSP by rspAb is an example of an antigen: antibody affinity. Another highly specific molecular affinity is that of the receptor site on an enzyme molecule for its substrate. This affinity has never been exploited as a probe because (1) the substrate is often a small molecule which does not lend itself to labeling with a marker, (2) the substrate is rapidly converted by the enzyme into a product, and (3) the product is quickly released from the enzyme molecule. However, it has now been determined that a probe can be developed for certain enzyme/substrate affinities, such as with the RSP, where these conditions do not apply. In the absence of either magnesium ions or adenosine triphosphate (ATP), the specific receptor sites on the enzyme component of RSP can bind specific tRNA's, with or without their cognate amino acid, without forming a releasable product. The bound tRNA can be labeled with photobiotin which permits easy linkage of tRNA with alkaline phosphatase conjugated streptavidin. After such a probe selectively binds with its cognate synthetase structure in RSP, its presence can be demonstrated through visible color development by an alkaline phosphatase/substrate reaction. Examples of sensitive color development procedures for this enzyme are simultaneous formation of indigo and formazan.

In accordance with the present method, the presence of RSP is determined by collecting a biological fluid sample from the individual undergoing examination. Unlike prior methods, however, it doesn't matter, in the detection of RSP, what type of cancer is suspected or which biological fluid is used. After the biological fluid has been collected the RSP in the fluid is captured onto a substrate using an anti-RSP antibody selected from one or more of monoclonal, polyclonal and affinity purified antibodies, as described and illustrated in Examples I and II. Thereafter, the presence of RSP on the substrate is preferably detected using the tRNA probe described hereinafter. Alternatively, in accordance with another preferred form of the invention the RSP in the biological fluid is both captured onto the substrate and detected using the tRNA probe.

As illustrated diagrammatically in Figure 1, the tRNA probe comprises a tRNA specific to the aminoacyl transfer RNA synthetase reactive site on the RSP tumor marker. Desirably, the tRNA has been labeled for subsequent detection, as with photobiotin. The tRNA probe attaches to the aminoacyl transfer RNAse reaction site on the RSP. Detection of the tRNA tagged RSP is facilitated by linking streptavidin to the photobiotin labeled tRNA and alkaline phosphatase to the streptavidin. Finally, a color developer such as 5-bromo-4-chloro-3-indolylphosphate/nitro blue tetrazolium reaction mixture is reacted with the alkaline phosphatase on the tRNA probe to develop a distinctive color having an intensity correlated to the quantity of RSP released by the cancerous cells in the biological fluid. In particular, the formation of blue/violet color

in this biotin-streptavidin-alkaline phosphatase system is indicative of the presence of the RSP tumor marker and, therefore, of a malignancy. Quantitative determination is achieved by the intensity of the produced color. Thus, the negligible to small RSP production observed with some non-malignant cells is several orders of magnitude smaller than the RSP production observed with malignant cells and there is little difficulty, via visual colorimetric intensity comparisons, in distinguishing between negligible and significant RSP production. It is noteworthy that an rspAb probe can be formed in the very same manner as the tRNA probe herein described and can be schematically illustrated, as in Figure 1, except that the tRNA component of the probe there illustrated would be substituted by an rspAb.

Desirably, the RSP tumor marker is captured out of the biological fluid, preferably using rspAb, and is labeled and linked with appropriate secondary molecules for subsequently developing visible color by reacting the tumor marker, in a preferred embodiment of the invention, with a tRNA-photobiotin-streptavidin-alkaline phosphatase probe, as shown in Figure 1. However, in another embodiment, the tRNA probe is used in lieu of the rspAb for capture as well as for detection of the RSP by reacting the tumor marker with the tRNA-photobiotin-streptavidin-alkaline phosphatase probe. Whether rspAb or tRNA is used for capture of the RSP from the biological fluid, the tRNA probe is most effective and preferably utilized for detecting the presence of RSP. Thus, in a blind study involving blood serum from well characterized patients in a clinical setting, the tRNA probe correctly identified 71/73 cancer sera for a 97.3% sensitivity and 53/62 non-cancer sera for a 85.5% specificity.

One example of the use of the tRNA probe for detection of the RSP on a substrate is the embodiment of the invention wherein the tRNA probe is used for both capture and detection. Using a blood serum sample collected from a patient undergoing examination, an aliquot of serum is placed within a prepared microtiter well which is coated or pretreated along its floor and/or wall surfaces with tRNA. Due to the affinity between the tRNA and the aminoacyl transfer RNA synthetase reactive sites on the RSP, any RSP in the sample becomes attached to the tRNA-pretreated surfaces of the well. The well is rinsed several times with water or phosphate buffered saline (PBS), pH 7.4, for washing all of the blood serum sample from the well except for the RSP bound to the well surfaces. A quantity of tRNA probe, in accordance with the present invention, comprising linked molecules of tRNA-photobiotin-streptavidin-alkaline phosphatase, as shown in Figure 1, is added to the well wherein the tRNA molecule reacts with and links to additional aminoacyl transfer RNA synthetase reactive sites on the RSP in the well. The condition in the well just prior to and immediately following the reaction between the tRNA probe and the RSP is illustrated, respectively, in Figure 2 and 3. Figure 2 shows the RSP markers bound to the tRNA precoated onto the well floor and unreacted tRNA probes in accordance with the present invention immediately prior to attaching to the RSP reactive sites. Figure 3 illustrates the RSP markers following reaction and shows the tRNA probes linked to available reactive sites on the RSP. Following attachment of the tRNA probes to the RSP receptor sites, the well is again rinsed with water or PBS to wash unreacted probe from the well. At this juncture it remains only to provide a visual indication of the quantity of RSP in the well. This is accomplished in the photobiotin-streptavidin-alkaline phosphatase system by adding 5-bromo-4-chloro-3-indolyl Phosphate (BCIP)/nitro blue tetrazolium (NBT) color reaction mixture to the well. Depending upon the concentration of RSP within the well the color system will produce a visual color indication ranging from no color, where from none to undetectable quantities of RSP have been captured from the biological fluid, to a very strong blue/violet color, where significant quantities of RSP have been captured. A quantitative measure of the color intensity can be determined with a spectrophotometer. By providing the test administrator with numerical values which clearly indicate the threshold color intensity representing the presence of malignancy, it is relatively simple to evaluate the results of the RSP detection test.

Example III

An experiment was performed to (1) confirm that the ring shaped particles contained enzymes with dinucleotide folds, i.e., that they contained aminoacyl tRNA synthetases, and (2) the substrate, tRNA, selectively binds to that receptor site. An affinity column for the dinucleotide fold in proteins (those enzymes that bind nucleotides as substrates), containing Affigel Blue (100-200 mesh, BioRad Laboratories, Richmond, CA) was set up according to the procedure of Thompson, et al., (Proceedings of the National Academy of Science, USA, 72:669-672, 1975). Culture medium conditioned by the growth of PC3, human prostate carcinoma cells, and known to contain RSP, was passed through the column and eluted with phosphate buffered saline (PBS), pH 7.4. Two milliliter aliquots of the elution fluid were tested for their protein content, using the method of Bradford, (Analytical Biochemistry, 72:248, 1976). After all protein species without dinucleotide folds were flushed through, 1 mg/ml ($4X10^{-2}$ mM) tRNA was introduced into the column. A significant rise in protein content was recorded in subsequent elution fractions, indicating that

tRNA preferentially bound to the dinucleotide folds in the synthetase molecules and dissociated the RSP from the Affigel Blue column. This result could not have been obtained unless both conditions described above (a dinucleotide fold was present which showed an affinity for tRNA) existed.

Example IV

An experiment was performed to demonstrate that tRNA labeled with photobiotin and streptavidin-alkaline phosphatase, using the method of Mierendorf, (Promega Notes, 1988), would link with RSP bound to the floor of microtiter wells and could be detected as a blue/violet color change in the well.

Immulon microtiter wells coated along the well floor surface with tRNA (see Figure 2) were filled with 100 microliters of medium conditioned by PC3 prostatic carcinoma cells and concentrated four (4) fold with Amicon centricon 30 microconcentrator. The RSP in the medium was, therefore, concentrated four (4) fold. After 30 minutes the medium was removed and the wells were rinsed 10 times with phosphate buffered saline (PBS), pH 7.4. The RSP, by reaction with the tRNA, were thereby attached to the floor of the wells. Ten microliters of photobiotin labeled tRNA was added to the treatment wells while 10 microliters of Tris buffer, pH 7.4 without tRNA, was added to the negative control wells. After 30 minutes at room temperature, the wells were rinsed 5 times with PBS. In this step, the tRNA attached to the aminoacyl transfer RNAse reaction site (see Figure 3) while, in the Tris treated wells without tRNA, the reaction site remained unreacted.

Twenty-five microliters of 0.1 mg/ml streptavidin was added to all wells. After 30 minutes, at room temperature, the wells were rinsed 10 times with PBS. The streptavidin linked with the photobiotin on the tRNA but washed out of the wells without tRNA.

Twenty-five microliters of 0.1 mg/ml biotinylated alkaline phosphatase was added to all wells. After 30 minutes at room temperature, the wells were rinsed 10 times with PBS. In this step, the biotin-alkaline phosphatase molecules were linked to the streptavidin on the photobiotinized tRNA linked to the RSP on the floor of the wells. This step completed the probe (Figure 1). The alkaline phosphatase washed out of the wells without tRNA.

Finally, 100 microliters of 5-bromo-4-chloro-3-indolyl phosphate (BCIP)/nitro blue tetrazolium (NBT) color reaction mixture was added to each well. Color developed in 45 to 60 minutes. The RSP treated with tRNA developed a strong blue-violet color reaction while the negative control remained unstained.

This study demonstrated conclusively that RSP has a reactive site specific for tRNA and that the tRNA probe, as described, will bind with and accurately identify RSP. As such, the probe offers a unique method for detecting malignancies in humans and animals.

Another desirable method of detecting the RSP is by use of a second anti-RSP antibody ($rspAb^2$) which is linked to the attached RSP:rspAb resulting from capturing the RSP from a body fluid using a rspAb, as hereinbefore described. According to this detection method a solid state substrate-rspAb:RSP:$rspAb^2$ sandwich is made in which the $rspAb^2$ antibody is used to agglutinate or precipitate the sandwiched molecules. The precipitate is filtered out with clear polycarbonate filters and the amount of precipitate is then measured by comparison to a standard color chart developed with RSP spiked samples or is quantitated by measurement with a differential reflecting spectrometer. One solid state substrate which is useful is colored (black) latex beads which produce a precipitate in shades of black depending on the amount of RSP present in the serum. An example of the practice of this method is set forth below:

a) rspAb are bound to carboxylated black latex beads (O), e.g. = O-rspAb.

b) The O-rspAb is reacted with the test serum in microcentrifuge tubes for a period of time after which the beads are centrifuged out of the serum. The beads are washed several times with PBS (phosphate buffered saline) and centrifuged out of the PBS after each wash. The resulting reactions are:

cancer sera = O-rspAb:RSP
non-cancer sera = O-rspAb

c) The reacted beads are then reacted with $rspAb^2$ in the microcentrifuge tubes for a period of time after which the beads are centrifuged out of the reaction mixture and the unbound molecules are washed out as described in b) above. The resulting reactions are:

cancer sera = O-rspAb:RSP:rspAb$^2$ (precipitate)

non-cancer sera = O-rspAb (no precipitate)

d) The precipitate is filtered through a clear polycarbonate filter membrane and a measurement is made. The non-precipitated latex beads flow through the membrane but the precipitated latex bead-antibody:RSP:antibody aggregates do not. Therefore, the beads can be separated from the precipitate very easily.

While the method and system illustrated hereinabove utilizes colored latex beads, it will be appreciated that a colored precipitate, fluorescent precipitate or radioactive precipitate could also be utilized by labeling the rspAb$^2$ with the appropriate molecules (e.g., any intensely colored particle such as formazan, carbon, etc.; fluorescent molecules such as rhodamine, Texas red, ethidium bromide, fluorescine, etc.; and, isotopes such as $^{125}I$, $^{3}H$, $^{14}C$, etc.). In another variation of the method and system illustrated hereinabove, the rspAb need not be linked to a solid state substrate and the rspAb:RSP:rspAb$^2$ or, simply, the rspAb:RSP reacted molecules could be precipitated out of the serum with an anti-rspAb antibody. Any of the indicator systems referred to previously herein could be on any one of the antibodies. Otherwise, the washing, filtering and quantitation procedures would be the same as described hereinabove.

While all of the biochemical properties of the RSP have not yet been deciphered, it is anticipated that each of the segments that make up the RSP has aminoacyl tRNAse activity for a specific amino tRNA:amino acid combination. Therefore, any of the tRNAs or any combination of tRNAs could be used to be made into the probe of the present invention. Moreover, either the same or different tRNAs can be used in the probe and along tRNA pretreated surfaces of the reaction vessel. Further, it is clear that the probe could easily be made with commonly used detection systems other than the biotin-streptavidin-alkaline phosphate-color system. For example, the tRNA could be linked to fluorescent markers such as fluorescein, rhodamine, Texas red, ethidium bromide, acridine dyes, etc. and be read with a spectrofluorometer, fluorescent microscope, or ultraviolet light spectrophotometer. The tRNA could also be linked with any of the radioactive isotopes and be detected with a beta or gamma counter or with autoradiography depending on the isotopes used. Furthermore, while the system described in which color development occurs in the liquid phase of the reaction vessel offers the most sensitivity, the RSP-tRNA probe detection system can also be adapted to solid substrate configurations such as membranes, glass, various polymer surfaces, latex beads and red blood cells (in agglutination tests), and metals such as colloidal gold or other enzymatic systems such as acid phosphatase, horse radish peroxidase, glucose oxidase and urease based systems.

## Industrial Applicability

The methods and probes of the present invention are broadly applicable to providing widespread screening for early cancer detection, a simple monitoring system for cancer patients undergoing treatment and a greatly improved method of screening for environmental and industrial carcinogens. Most significant, perhaps, is the ability to utilize the methods to assemble RSP probes into a test kit which offers physicians a quick, sensitive, reliable and inexpensive system for the detection of RSP in body fluids of individuals during routine physical examinations or during the treatment of cancer patients following radiation or chemotherapy. If a positive test result is present in an apparently healthy individual, a repeat test would be warranted. If the second test confirmed the presence of RSP, further tests could be undertaken (e.g., NMR Imaging) to locate and identify the tumor in the body. At that point a course of therapy would be recommended to remove or destroy the malignant tissue and the RSP could be used to monitor the success of the therapy.

The availability of a single, easy-to-use, in vitro diagnostic test for carcinogenic activity anywhere in a patient's body is expected to make possible early detection of cancerous activity and accurate monitoring of cancer treatment. When it is appreciated that there are at least five million people with a history of cancer who should be tested quarterly and an additional few million newly diagnosed and active cases each year, the magnitude of the need for a quick, effective diagnostic test becomes apparent. Additionally, the availability of a biological fluid test in lieu of the presently uncomfortable, often embarrassing "Pap" smear test would likely encourage currently reluctant women to take the RSP test on at least an annual basis, thus further evidencing the need for and usefulness of such a test.

## Claims

1. A method for detecting the presence of the ring shaped particle (RSP) tumor marker in biological fluids comprising the steps of:
   a. capturing the RSP tumor marker in said fluid onto a substrate; and
   b. detecting the presence of the RSP tumor marker on said substrate.

2. A method for detecting the presence of malignancy in humans or animals comprising the steps of:
   a. obtaining a body fluid sample from a human or animal to be examined for the presence of a malignancy;
   b. capturing the ring shaped particle (RSP) tumor marker in said fluid onto a substrate; and
   c. detecting the presence of the RSP tumor marker on said substrate.

3. A method, as claimed in claims 1 or 2, wherein said step of capturing the RSP tumor marker in said fluid comprises attaching to said RSP an anti-RSP antibody (rspAb) selected from the group consisting of monoclonal antibodies, polyclonal antibodies, affinity purified antibodies, and mixtures thereof.

4. A method, as claimed in claims 1 or 2, wherein said step of capturing the RSP tumor marker in said fluid comprises attaching, in the absence of at least one of magnesium ions and adenosine triphosphate (ATP), transfer ribonucleic acid (tRNA) specific to the aminoacyl transfer RNA synthetase reactive site on said marker.

5. A method, as claimed in claims 3 or 4, wherein said step of detecting the presence of the RSP tumor marker comprises developing visible color as an indicator of the presence of the RSP tumor marker.

6. A method, as claimed in claim 5, including the step of directly or indirectly linking at least one color reactive molecule to the RSP tumor marker for developing said visible color.

7. A method, as claimed in claim 3, wherein said step of detecting the presence of the RSP tumor marker comprises attaching, in the absence of at least one of magnesium ions and adenosine triphosphate (ATP), transfer ribonucleic acid (tRNA) specific to the aminoacyl transfer RNA synthetase reactive site on said marker and detecting the presence of the tRNA as an indicator of the presence of the RSP tumor marker.

8. A method, as claimed in claims 4 or 7, wherein said tRNA has at least one color reactive secondary molecule associated therewith for developing visible color as an indicator of the presence of the RSP tumor marker.

9. A method, as claimed in claim 8, including the step of reacting said color reactive secondary molecule with a color developer for forming a distinctive color indicating the presence of the RSP tumor marker.

10. A method, as claimed in claim 8, wherein said tRNA is attached to said marker by reacting with said marker a tRNA probe including tRNA and at least one color reactive secondary molecule for developing visible color as an indicator of the presence of the RSP tumor marker.

11. A method, as claimed in claim 10, wherein said tRNA probe comprises the linked molecules tRNA-photobiotin-streptavidin-alkaline phosphatase.

12. A method, as claimed in claim 11, including the step of reacting said RSP-attached tRNA probe with a 5-bromo-4-chloro-3-indolyl phosphate/nitro blue tetrazolium reaction mixture for forming a distinctive blue/violet color indicating the presence of the RSP tumor marker.

13. A method, as claimed in claim 3, wherein said step of detecting the presence of the RSP tumor marker comprises linking to the RSP:rspAb molecule a second anti-RSP antibody ($rspAb^2$) selected from the group consisting of monoclonal antibodies, polyclonal antibodies, affinity purified antibodies, and mixtures thereof.

14. A probe for selectively binding to the RSP tumor marker for facilitating the detection of its presence in a biological fluid comprising a first probe element adapted to attach or associate with said tumor

marker and a probe marker linked to said first probe element, the presence of said probe marker being adapted to be detected as an indicator of the presence of the RSP tumor marker.

**15.** A probe, as claimed in claim 14, wherein said first probe element comprises an anti-RSP antibody selected from the group consisting of monoclonal antibodies, polyclonal antibodies, affinity purified antibodies, and mixtures thereof.

**16.** A probe, as claimed in claim 14, wherein said first probe element comprises tRNA specific to the aminoacyl transfer RNA synthetase reactive site on said marker.

**17.** A probe, as claimed in claims 14, 15 or 16, wherein said first probe element is linked directly or indirectly to at least one color reactive secondary molecule adapted for developing visible color as an indicator of the presence of the RSP tumor marker.

**18.** A probe, as claimed in claim 17, wherein said probe marker comprises the linked molecules tRNA-photobiotin-streptavidin-alkaline phosphatase.

**19.** A probe, as claimed in claims 14, 15 or 16, wherein said first probe element is linked directly or indirectly to secondary molecules adapted for developing fluorescence as an indicator of the presence of the RSP tumor marker.

**20.** A probe, as claimed in claims 14, 15 or 16, wherein said first probe element is linked directly or indirectly with a radioactive isotope for indicating the presence of the RSP tumor marker.

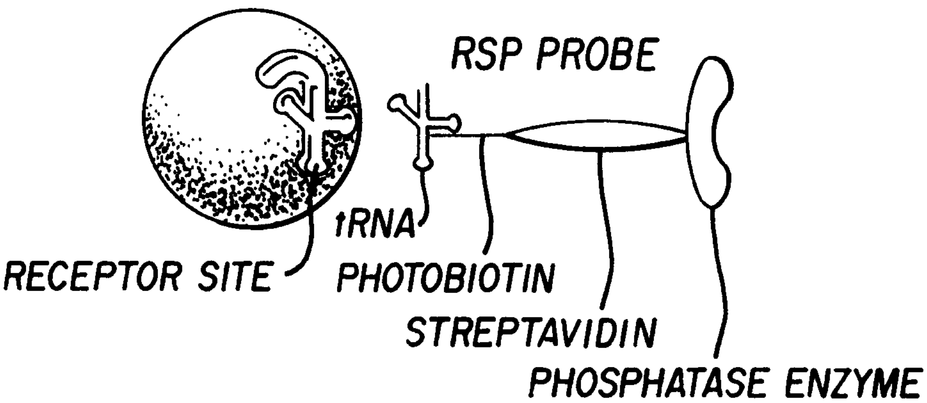
RSP PROBE
tRNA
RECEPTOR SITE
PHOTOBIOTIN
STREPTAVIDIN
PHOSPHATASE ENZYME

FIG. 1

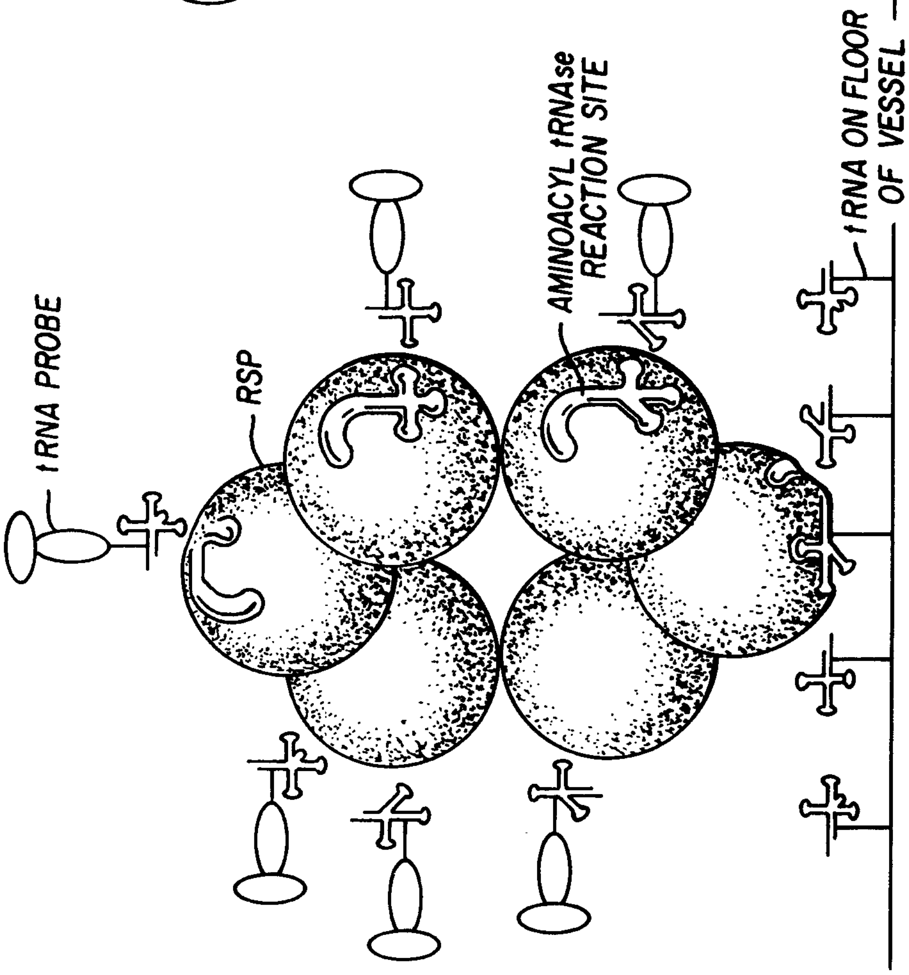
tRNA PROBE
RSP
AMINOACYL tRNAse REACTION SITE
tRNA ON FLOOR OF VESSEL

FIG. 2

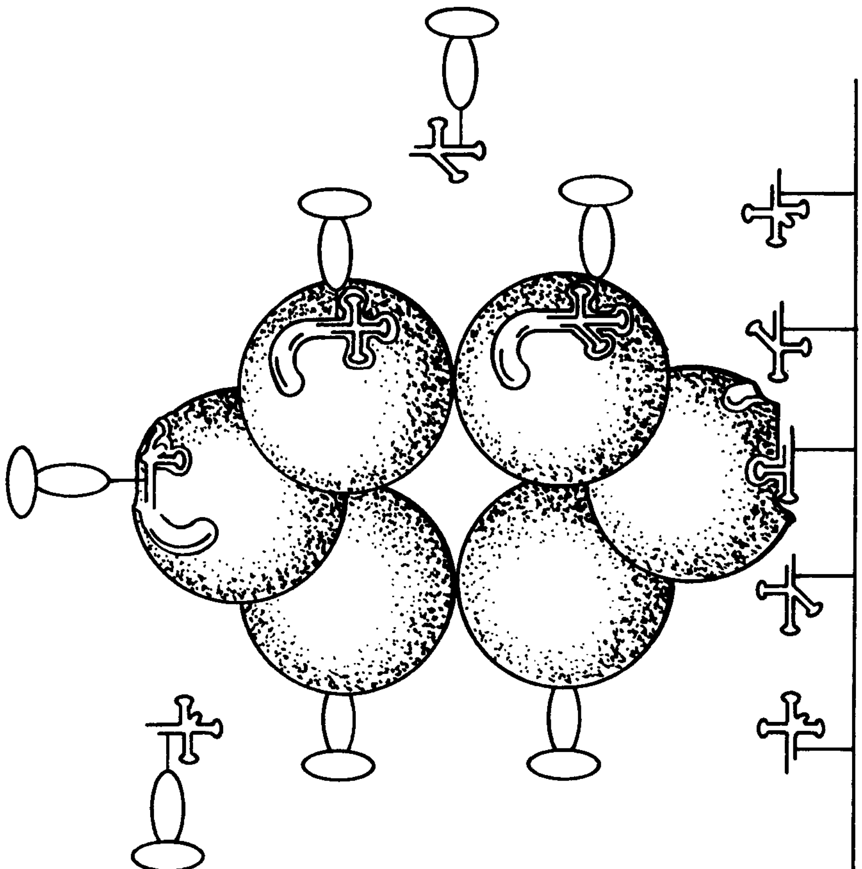

FIG. 3

European Patent Office

# EUROPEAN SEARCH REPORT

Application Number

EP 90 11 5425

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | SCHWEIZ. MED. WSCHR., vol. 119, no. 39, 1989, pages 1342-1343; A.J. WIECZOREK et al.: "Ein Gensondentest für RNA-Proteolipid in Serumproben bei Neoplasie"<br>* Whole document * | 1,2 | G 01 N 33/574<br>C 12 Q 1/68<br>G 01 N 33/58 |
| X | FR-A-2 586 814 (CENTRE NATIONAL DE LA RECHERCHE SCIENTIFIQUE (CNRS))<br>* Page 1, line 1 - page 2, line 36; page 7, line 6 - page 11, line 30 * | 14,15, 17,19, 20 | |
| X | GB-A-2 067 286 (BAYLOR COLLEGE OF MEDICINE)<br>* Page 1, line 1 - page 3, line 86; page 7, lines 60-95 * | 14,15, 17,19, 20 | |
| X | CANCER INVESTIGATION, vol. 1, no. 1, 1983, pages 25-40; H. BUSCH et al.: "Ultrastructural and purification studies on human tumor nucleolar antigens and nucleolar paricles"<br>* Whole document * | 14,15, 17,19 | TECHNICAL FIELDS SEARCHED (Int. Cl.5) |
| A | IDEM | 1-3 | G 01 N<br>C 12 Q |
| X | THE EMBO JOURNAL, vol. 9, no. 6, June 1990, pages 1757-1767; J.M. PETERS et al.: "An abundant and ubiquitous homo-oligomeric ring-shaped ATPase particle related to the putative vesicle fusion proteins Sec 18p and NSF"<br>* Abstract; pages 1760-1761 *<br>-/- | 14,15,1 ,7,19 | |

The present search report has been drawn up for all claims

| Place of search | Date of completion of the search | Examiner |
|---|---|---|
| THE HAGUE | 17-10-1990 | HITCHEN C.E. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

Application Number

EP 90 11 5425

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | CANCER RESEARCH, vol. 47, 1st December 1987, pages 6407-6412; A.J. WIECZOREK et al.: "Diagnostic and prognostic value of RNA-proteolipid in sera of patients with malignant disorders following therapy: First clinical evaluation of a novel tumor marker" * Abstract * --- | 1 | |
| A | GYNECOLOGIC ONCOLOGY, vol. 4, 1976, pages 125-132; D.E ROUNDS et al.: "Prospects for a personal screening method for cervical carcinoma" * Abstract * ----- | 1 | |
| | | | TECHNICAL FIELDS SEARCHED (Int. Cl.5) |

The present search report has been drawn up for all claims

| Place of search | Date of completion of the search | Examiner |
|---|---|---|
| THE HAGUE | 17-10-1990 | HITCHEN C.E. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document